(19) Europäisches Patentamt
European Patent Office
Office européen des brevets

(11) **EP 4 614 504 B1**

(12) **EUROPEAN PATENT SPECIFICATION**

(45) Date of publication and mention
of the grant of the patent:
**29.04.2026 Bulletin 2026/18**

(21) Application number: **24161074.0**

(22) Date of filing: **04.03.2024**

(51) International Patent Classification (IPC):
**G16H 20/17** (2018.01)   **G16H 40/63** (2018.01)
**G16H 50/30** (2018.01)   **G16H 50/70** (2018.01)
**G16H 50/20** (2018.01)

(52) Cooperative Patent Classification (CPC):
**G16H 20/17; G16H 40/63; G16H 50/20;
G16H 50/30; G16H 50/70**

(54) **COMPUTER-IMPLEMENTED METHOD FOR PREDICTING A RISK WHETHER A HYPOGLYCEMIC EVENT WILL OCCUR, DATA PROCESSING SYSTEM, COMPUTER PROGRAM, SYSTEM, AND REMOTE CONTROL**

COMPUTERIMPLEMENTIERTES VERFAHREN ZUR VORHERSAGE EINES RISIKOS, OB EIN HYPOGLYKÄMISCHES EREIGNIS AUFTRITT, DATENVERARBEITUNGSSYSTEM, COMPUTERPROGRAMM, SYSTEM UND FERNSTEUERUNG

PROCÉDÉ MIS EN OEUVRE PAR ORDINATEUR POUR PRÉDIRE UN RISQUE D'APPARITION D'UN ÉVÉNEMENT HYPOGLYCÉMIQUE, SYSTÈME DE TRAITEMENT DE DONNÉES, PROGRAMME INFORMATIQUE, ET TÉLÉCOMMANDE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**10.09.2025 Bulletin 2025/37**

(73) Proprietor: **Roche Diabetes Care GmbH
68305 Mannheim (DE)**

(72) Inventors:
• **RINGEMANN, Christian
68305 Mannheim (DE)**
• **KLOPFENSTEIN, Yannick
8048 Zürich (CH)**
• **LEPPÄAHO, Eemeli
00530 Helsinki (FI)**
• **LUSTENBERGER, Patrick
8048 Zürich (CH)**
• **PEAK, Ajandek
80807 München (DE)**

(74) Representative: **Bittner, Thomas L.
Boehmert & Boehmert
Anwaltspartnerschaft mbB
Pettenkoferstrasse 22
80336 München (DE)**

(56) References cited:
**WO-A1-2019/157102     US-A1- 2020 375 549**

• **DAVE DARPIT ET AL: "Feature-Based Machine Learning Model for Real-Time Hypoglycemia Prediction", JOURNAL OF DIABETES SCIENCE AND TECHNOLOGY, vol. 15, no. 4, 1 June 2020 (2020-06-01), US, pages 842 - 855, XP093169979, ISSN: 1932-2968, DOI: 10.1177/ 1932296820922622**
• **VU LONG ET AL: "Predicting Nocturnal Hypoglycemia from Continuous Glucose Monitoring Data with Extended Prediction Horizon", 4 March 2020 (2020-03-04), XP093171143, Retrieved from the Internet <URL:https://www.ncbi.nlm.nih.gov/pmc/ articles/PMC7153099/pdf/3200473.pdf>**

## Description

The present disclosure refers to a computer-implemented method for predicting a risk whether a hypoglycemic event will occur, a data processing system, a computer program, a (CGM) system, and a remote control.

Background

Past (blood) glucose values, insulin intakes, or carbohydrate values are known to influence future glucose values including the possibility to fall below a certain glucose threshold constituting a hypoglycemic event. The difficulty typically lies in obtaining a suitable modelling and aggregation and of the past values for specific prediction tasks.

Document US 11,350,876 B2 pertains to a patient health management platform which determines a metabolic state for a first time period. The platform generates a patient-specific treatment recommendation for a second time period following the first time period that identifies objectives for the patient to complete to improve the metabolic state determined for the first time period.

In document US 2022 / 0 061 710 A1, a patient health management platform implements a machine-learned metabolic model to generate a prediction of a patient's glucose level. The platform implements a short-term prediction model to generate a daily prediction of the patient's glucose level based on nutrition data reported by the patient and sensor data and lab test data collected for the patient.

Document US 2022 / 0 142 521 A1 discloses a method of predicting an analyte concentration trend to provide a user with an opportunity to take therapeutic measures if needed. The method includes receiving a plurality of past measured analyte concentrations between a time of a most recent measured analyte concentration and a time of an earlier measured analyte concentration; calculating a data set comprising differences in measured analyte concentrations between consecutive measured analyte concentrations; and predicting whether a hypo/hyper analyte concentration event will occur within a predetermined time period.

In document US 2021 / 0 338 116 A1, hypoglycemic event prediction using machine learning is described. A CGM platform includes a machine learning model trained using historical time series glucose measurements of a user population. Once trained, the machine learning model predicts hypoglycemic events for users. When predicting hypoglycemic events, a time series of glucose measurements for a day time interval is received. The glucose measurements of this time series for the day time interval are provided by a CGM system worn by the user. The machine learning model predicts whether a hypoglycemic event will occur during a night time interval that is subsequent to the day time interval by processing the time series of glucose measurements using the trained machine learning model.

In document US 2020 / 375 549 A1, a computer-implemented method for forecasting a blood glucose state of a patient is disclosed. The method comprises receiving blood glucose data and generating an initial prediction of the blood glucose state by applying a first set of machine learning models. The method further comprises determining features from the initial prediction; and generating a final prediction of the blood glucose state by applying a second set of machine learning models.

Summary

It is an object of the present disclosure to provide a robust method with improved predictability of the risk of experiencing a hypoglycemic event.

For solving the problem, a computer-implemented method for predicting a risk whether a hypoglycemic event will occur during a prediction time interval according to independent claim 1 is provided. Moreover, a data processing system according to claim 14 and a computer program according to claim 15 are provided. Further embodiments are disclosed in dependent claims.

According to one aspect, a computer-implemented method for predicting a risk whether a hypoglycemic event will occur during a prediction time interval is provided. The method comprises:

- providing user values for a plurality of machine learning input parameters, the user values determined from glucose monitoring data (with glucose measurement values) detected for a user before a cut-off time, the plurality of machine learning input parameters comprising: at least one first glucose measurement value determined from glucose measurements values measured within at most 10 minutes before the cut-off time, a hypoglycemic event share value indicative of a fraction (proportion) of previous prediction time intervals with detected hypoglycemic events and a total number of previous prediction time intervals before the cut-off time, and a first hypoglycemic event number value being a first number of hypoglycemic events detected within the previous prediction time intervals within a number of days before the cut-off time ranging from 7 days to 21 days;
- predicting, based on the user values and using a machine learning model, a risk whether a hypoglycemic event will occur during a prediction time interval (after the cut-off time) (associated with (assumed) sleep of the user), the machine learning model generated based on the machine learning input parameters and using historical glucose monitoring data of a user population; and

- generating an output based on the predicted risk.

According to another aspect, a data processing system is provided. The data processing system comprises means for carrying out the computer-implemented method. In particular, the data processing system may comprise a processor unit and/or a memory unit and/or a communication unit.

According to still another aspect, a computer program (product) is provided. The computer program comprises instructions which, when the computer program is executed by the data processing system, cause the data processing system to carry out the computer-implemented method.

In accordance with another aspect, a (CGM) system is provided comprising a (continuous) glucose measurement sensor device, a remote control configured to receive glucose data from the continuous glucose measurement sensor device, and a processing unit configured to carry out the computer-implemented method.

A further aspect concerns a remote control configured to generate an output and comprising a processor configured to carry out the computer-implemented method. The remote control may be configured to control the (continuous) glucose measurement device and/or an insulin pump.

The present method allows for providing the user prior to going to sleep with a reliable and actionable prediction of the risk of experiencing a hypoglycemic event within the prediction time interval. Thus, the user may be enabled to take preventive actions such as consuming carbohydrates or reducing a basal insulin intake. In particular, the method may make use of certain machine learning input parameters (also known in the art of machine learning as (machine learning) features) which already contain an adequate amount of information for obtaining probabilities for hypoglycemic events. Hence, a simplified and targeted data collection is made possible while preserving an adequate model performance. Further, using the machine learning input parameters, overfitting may be reduced and hence the robustness of the prediction results be increased.

The method may be a method for predicting a risk whether a hypoglycemic event will occur for a user (person having diabetes). The hypoglycemic event may correspond to glucose level below 70 mg/dL.

The data processing system may comprise at least one of a user device such as a personal data processing device, in particular of the user / the person having diabetes, an input device, and an output device. Additionally, or alternatively, the data processing system may comprise a glucose monitoring device and/or a glucose monitoring system and/or a medical server. The data processing system may be one of the user device, the glucose monitoring device/-system, and the medical server.

The glucose monitoring data may further comprise at least one of measurement time values (i.e., times at which the glucose measurement values have been measured and/or detected), carbohydrate intake values, carbohydrate intake times, (bolus) insulin intake values, e.g., provided by an insulin pump or a pen, and insulin intake times.

The method may comprise detecting the glucose monitoring data, in particular within an acquisition time interval before the cut-off time, preferably by the glucose monitoring device and/or glucose monitoring system. Detecting the glucose monitoring data may comprise measuring and/or detecting and/or determining of at least one of the glucose measurement values, the measurement time values, the insulin intake values, and the insulin intake times.

The method may further comprise transmitting the glucose monitoring data (at least partially) from the glucose monitoring device/system to the data processing system, in particular to at least one of the user device, the input device, and the medical server. The method may comprise providing, in particular: receiving, the glucose monitoring data, in particular including the glucose measurement values of the user measured within an acquisition time interval before the cut-off time, preferably in at least one of the user device, the input device, and the medical server.

The glucose monitoring data, in particular, at least one of the glucose measurement values, the carbohydrate intake values, and the insulin intake values, may (at least partially) be provided within time bins, i.e., within time intervals having a length between 30 second and 10 minutes, preferably between 2 minutes and 6 minutes, more preferably 5 minutes. Preferably the glucose monitoring data cover consecutive time bins. To this end, measured (raw) glucose measurement values within one glucose value time interval may be aggregated, in particular averaged, e.g., using an arithmetic mean of the (raw) glucose measurement values within the one glucose value time interval. The carbohydrate intake values and/or the insulin intake values may be aggregated by summing up the carbohydrate intake values / insulin intake values within the respective time bin.

The method may comprise determining (at least partially) the user values for the plurality of machine learning input parameters from the glucose monitoring data, preferably in the data processing system, in particular in at least one of the user device, the glucose monitoring device, and the medical server. The user values for the plurality of machine learning input parameters may be provided in at least one of the user device, the glucose monitoring device, and the medical server. Providing the user values may comprise (at least partially) receiving the user values and/or (at least partially) determining the user values, in particular from the glucose monitoring data.

Generating the machine learning model may comprise training the machine learning model based on the machine learning input parameters and using the historical glucose measurement values of the user population for the machine learning input parameters. The machine learning model may have been generated in the data processing system, in particular in at least one of the user device, the glucose monitoring device, and the medical server. The machine learning model may have been generated based on a glucose measurement value resolution between 0.1 mg/dL and 5 mg/dL, preferably at 0.5 mg/dL or at 1 mg/dL.

The risk may be predicted in the data processing system, in particular in at least one of the user device, the glucose monitoring device, and the medical server. The output may be generated in the data processing system, in particular in at least one of the user device, the glucose monitoring device, and the medical server. The prediction time interval may be after / start with the cut-off time. The risk may be predicted at the cut-off time. Providing the user values in the user device and predicting the risk in the user device may allow for reliable risk prediction without or a poor network connection to a medical server.

Preferably, the steps of providing, in particular determining, the user values and predicting the risk may be carried out in the medical server and the output be displayed by the user device.

The cut-off time may be predefined and/or set by the user. The cut-off time may also be set in reaction to the user initiating/requesting a risk prediction, e.g., via the user device.

The at least one first glucose measurement value may be determined from glucose measurements values measured within at most 7 minutes before the cut-off time, preferably within at most 5 minutes before the cut-off time, more preferably within 5 minutes before the cut-off time.

The at least one first glucose measurement value may be single value (a first glucose measurement value measured within 10 minutes before the cut-off time) or an average value corresponding to a mean, in particular an arithmetic mean, of multiple first glucose measurement values, preferably each of which has been measured within 5 minutes or 7 minutes before the cut-off time.

The previous prediction time intervals may be selected from a set of days ranging from at most 21 days to at most 35 days before the day of the cut-off time, preferably ranging from at most 24 days to at most 32 days before the day of the cut-off time, more preferably of (at most) 28 days before the cut-off time.

The previous prediction time intervals may each be selected from the set of days depending on comprising at least a minimum number of glucose measurement values. For example, one of the previous prediction time intervals may be selected if it comprises at least 80 %, preferably at least 90 % of a maximum number of glucose measurement values. The maximum number of glucose measurement values may be between 50 and 200, preferably between 100 and 180, more preferably at 144.

The hypoglycemic event share value may be a fraction of a number of previous prediction time intervals with detected hypoglycemic events $n_h(t)$ and the total number of previous prediction time intervals $n_v(t)$. Hence, the hypoglycemic event share value may be determined as $n_h(t)/n_v(t)$.

More generally, the hypoglycemic event share value may be a fraction wherein the numerator comprises the number of previous prediction time intervals with detected hypoglycemic events and the denominator comprises the total number of previous prediction time intervals. The hypoglycemic event share value may be determined using a Bayesian posterior probability. For example, the hypoglycemic event share value may determined using the formula

$$\text{SLIDING\_EVENT\_HYPO\_L1\_BASELINE\_28D\_WP}(t) = \frac{RV8\_3 + n_h(t)}{RV8\_3 + RV8\_4 + n_v(t)}$$

with constants $RV8\_3$ and $RV8\_4$ , which may preferably be a beta distribution shape parameter and a rate parameter, respectively. In particular, the hypoglycemic event share value may determined by the formula:

$$\text{SLIDING\_EVENT\_HYPO\_L1\_BASELINE\_28D\_WP}(t) = \begin{cases} \dfrac{RV8\_3 + n_h(t)}{RV8\_3 + RV8\_4 + n_v(t)}, & \text{if } n_v(t) \geq RV8\_2 * 28\,/100 \\ DEF8, & \text{else} \end{cases}$$

**with constants** $DEF8, RV8\_2, RV8\_3$ and $RV8\_4$.

The total number of previous prediction time intervals $n_v(t)$ may be determined via the formula

$$n_v(t) = \sum_{i=1}^{m} (1 \text{ if nighttime range}(t - i \text{ days}) \text{ is valid, else } 0)$$

with m being a maximum number of days before the cut-off time, which may preferably be between 21 and 35, more preferably be 28. Herein, a nighttime range (prediction time interval) is *valid* if more than 90 % of the maximum number of glucose measurement values in the prediction time interval are available, in particular, assuming the glucose measurement values are provided in discrete time intervals.

The number of previous prediction time intervals with detected hypoglycemic events $n_h(t)$ may be determined via the formula

$$n_h(t) = \sum_{i=1}^{m} (1 \text{ if nighttime range } (t - i \text{ days})$$

is valid and has at least one hypoglycemic event, else 0) with m being the maximum number of days before the cut-off time.

Each of the detected hypoglycemic events $n_h(t)$ may be a level 1 hypoglycemic event, corresponding to glucose measurement values being below 70 mg/dL, in particular for at least 15 minutes.

The plurality of machine learning input parameters may comprise the first hypoglycemic event number value being a (first) number of hypoglycemic events, in particular, level 1 hypoglycemic events, detected within the previous prediction time intervals within the number of days before the cut-off time, preferably the number of days being 14.

Additionally or alternatively, the plurality of machine learning input parameters may comprise a second hypoglycemic event number value being a (second) number of hypoglycemic events, in particular, level 1 hypoglycemic events, detected within a detection time interval before the cut-off time having a length of at least 12 hours and at most 48 hours, preferably at least 18 hours and at most 30 hours, more preferably 24 hours.

The plurality of machine learning input parameters may further comprise a (first) insulin on board (IOB) value predicted for an insulin on board target time after the cut-off time. The insulin on board target time may be between 1 hour and 3 hours, preferably be 2 hours after the cut-off time.

The insulin on board value may be determined from at least one bolus insulin intake at or before the cut-off time, preferably using an insulin on board formula indicative of an insulin on board decline in time after a bolus insulin intake. The insulin on board formula may comprise at least one exponential decay term and/or at least one linear decay term. For example, the insulin on board value at time t may be determined by the following insulin on board formula:

$$\text{IOB}(t) = \left\{ 1 - s(1-a) \left( \left( \frac{t^2}{\tau t_d (1-a)} - \frac{t}{\tau} - 1 \right) e^{-\frac{t}{\tau}} + 1 \right) \right\} \cdot I_0$$

with $t_d$ : activity duration (acting time), $[t_d]$ = min, $t_p$ : peak activity time (offset time), $[t_p]$ = min, $\tau = t_p(1 - \frac{t_p}{t_d})/(1 - 2\frac{t_p}{t_d})$ : a time constant of exponential decay, $a = \frac{2\tau}{t_d}$ : rise time factor, $s = \frac{1}{1-a+(1+a)e^{-\frac{t_d}{\tau}}}$ : auxiliary scale factor, and $I_0$: insulin injected (bolus insulin intake, insulin intake amount) at insulin intake time $t = 0$.

In case of a plurality of insulin intakes, the insulin on board value may be determined from a (linear) superposition of insulin on board formulae, in particular with correspondingly shifted insulin intake times *t*. The method may comprise providing at least one insulin intake time and at least one insulin intake amount, e.g., in the data processing system, in particular in at least one of the user device, the glucose monitoring device, and the medical server. The at least one insulin intake time and at least one insulin intake amount may be transmitted from an insulin pump and/or input by the user.

The plurality of machine learning input parameters may further comprise a first slope value indicative of a change in time of a first set of glucose measurement values which have been measured within a first slope time interval before the cut-off time. The first slope time interval may have a length between 5 and 15 minutes, preferably of 10 minutes.

The plurality of machine learning input parameters may further comprise a second slope value indicative of a change in time of a second set of glucose measurement values which have been measured within a second slope time interval before the cut-off time. The second slope time interval may have a length between 20 and 45 minutes, preferably of 30 minutes.

The first slope value and/or the second slope value may be determined using linear regression, in particular, linear least-squares regression, for the glucose measurement values within the first (second) set. For example, the first slope value and/or the second slope value may be determined as:

$$\frac{N \sum_{i=1}^{N} x_i y_i - \sum_{i=1}^{N} x_i \sum_{i=1}^{N} y_i}{N \sum_{i=1}^{N} x_i^2 - \left( \sum_{i=1}^{N} x_i \right)^2},$$

with measurement times $x_i$, glucose measurement values $y_i$ and the number of measurements $N$. The first slope value and/or the second slope value may also be determined using a difference quotient.

The plurality of machine learning input parameters may comprise an unexpected glucose drop value being a linear combination of a change in time of glucose measurement values, which have been measured within a glucose drop time interval before the cut-off time, and a (second) insulin on board value at the cut-off time. The unexpected glucose drop value may be indicative of an unexpected drop of the glucose measurement values.

The unexpected glucose drop value may in particular be a sum of the change in time of glucose measurement values and the insulin on board value multiplied with an insulin on board factor, the insulin on board factor preferably being between 20 and 80, more preferably being between 40 and 60, more preferably being 50. The glucose drop time interval may have a length between 0.5 hours and 2 hours, preferably 1 hour.

The change in time of glucose measurement values can be determined as $\Delta CGM(t) = CGM(t) - CGM(t - l)$ with $l$ being the length of the glucose drop time interval. The unexpected glucose drop value at time $t$ may thus be determined as: $\Delta CGM_{fall}(t) = CGM(t) - CGM(t - 60 \text{ min}) + 50 \cdot IOB(t)$.

The plurality of machine learning input parameters may further comprise a shifted time value determined by shifting the cut-off time into the future by at least 2 hours and at most 5 hours (preferably by at least 2 hours and at most 4 hours, more preferably by at least 2.5 hours and at most 3.5 hours, more preferably by 3 hours) and rescaling, in particular linearly rescaling, the resulting value to a (float) interval, which preferably is [0, 24], wherein 0 corresponds to midnight (the midnight hour 0:00) and 24 to midnight next day (the midnight hour 24:00).

For example, for the cut-off time being 21:45, shifting by 3 hours results in the time 0:45, which corresponds to the shifted time value being 0.75 (= 45/60). For the cut-off time being 01:30, shifting by 3 hours results in the time 4:30, which corresponds to the shifted time value being 4.5 (= 4+30/60).

The plurality of machine learning input parameters may further comprise a carbohydrate consumption by the user within a carbohydrate consumption interval before the cut-off time. The length of the carbohydrate consumption interval may be between 0.5 hours and 4 hours, preferably between 2 hours and 3 hours.

The plurality of machine learning input parameters may have been determined out of a (greater) initial set of machine learning input parameters making, e.g., additionally use of a feature extraction method.

The prediction time interval and/or each of the previous time intervals may be a nighttime interval. For example, the prediction time interval and/or each of the previous time intervals may start at a prediction time interval starting time between 9 pm and 11 pm, preferably at 10 pm. Further, the prediction time interval and/or each of the previous time intervals may end at a prediction time interval ending time between 7 am and 9 am, preferably at 8 am.

The prediction time interval and each of the previous prediction time intervals may start and/or end at the same time of day. The cut-off time may be a time of day. The cut-off time may coincide with the prediction time interval starting time.

The prediction time interval and/or each of the previous time intervals may have a length between 5 hours and 10 hours, preferably between 6 hours and 8 hours, more preferably of 7 hours.

Predicting the risk may comprise predicting a first risk whether an early hypoglycemic event will occur during a first sub interval of the prediction time interval and/or predicting a second risk whether a late hypoglycemic event will occur during a second sub interval of the prediction time interval after the first sub interval. As a result, the predictability of hypoglycemic events may be improved and/or recommendations to the user may be more precise and adequate for the situation at hand. For example, many user actions may have a substantial metabolic effect for not more than 3 to 4 hours.

The first sub interval and/or the second sub interval may have a length between 2 hours and 5 hours, preferably of 3.5 hours.

The risk, the first risk, and the second risk may each indicate or be a probability value. The sum of the first risk, the second risk, and a probability of no hypoglycemic occurring may be (essentially) 1. The risk may be the sum of the first risk and the second risk. The sum of the risk and the probability of no hypoglycemic occurring may be (essentially) 1.

The output may comprise at least one of:

- the predicted risk and/or a risk signal indicating the predicted risk,
- a risk level determined from the predicted risk (and/or a risk level signal indicating the risk level),
- a time information indicating a time range within which the hypoglycemic event may occur,
- a recommendation, in particular to the user, indicating at least one user action to reduce the risk for a hypoglycemic event to occur during the prediction time interval and/or a recommendation signal indicating the recommendation, and
- a notification indicating that no further user action is required at the moment and/or a notification signal indicating the notification.

The user action may for example be at least one of eating a meal and/or carbohydrates, reducing the insulin intake, in particular reducing a basal rate of an insulin pump, and setting an alarm clock.

The method may comprise determining the risk level from the predicted risk. The risk level may indicate at least one of a normal risk, a high risk, and a very high risk. The risk level may be determined from the predicted risk by assigning the risk level based on the magnitude of the predicted risk. For example, the risk level may indicate a very high risk if the predicted risk is greater than or equal to a very high risk threshold. Further, the risk level may indicate a high risk if the predicted risk is greater than or equal to a high risk threshold and less than the very high risk threshold. The risk level may indicate a normal risk if the predicted risk is less than the high risk threshold. The very high risk threshold may be between 0.5 and 0.7, preferably be 0.6. The high risk threshold may be between 0.2 and 0.4, preferably be 0.3.

It may be provided that a notification, e.g., on the user device, is only generated in case a high risk or a very high risk is determined.

The method may comprise generating the recommendation based on the predicted risk. The recommendation may depend on whether an early hypoglycemic event or a late hypoglycemic event is predicted. For example, in case of a predicted early hypoglycemic event, the (recommended) user action may be eating a meal. The metabolic effect of eating the meal may not be sufficient for reducing the risk of a late hypoglycemic event. Thus, in case of a predicted late hypoglycemic event, the (recommended) user action may be setting an alarm clock.

Generating the output may comprise displaying the output, in particular indicating the predicted risk, in particular to the user. The output may for example be displayed on a display of the data processing system, in particular of at least one of the user device, the glucose monitoring device, and the medical server. Additionally or alternatively, generating the output may comprise transmitting the output (in particular, at least one of the risk signal, the risk level signal, the recommendation signal, and the notification signal) from at least one of the user device, the glucose monitoring device, and the medical server to at least another one of the user device, the glucose monitoring device, and the medical server. For example, generating the output may comprise transmitting the output from the medical server to the user device and subsequently displaying the output by the user device.

The method may comprise storing prediction information indicative of at least one of the predicted risk, the risk level, the recommendation, the notification, the cut-off time, a user identifier, the plurality of machine learning input parameters, error data, warning data, log data, and service data. The prediction information may be stored in at least one of the user device, the glucose monitoring device, and the medical server.

The machine learning model may have been generated using at least one of gradient boosting (e.g., XGBoost), a decision tree, a random forest, a logistic regression, and a artificial neural network, in particular, a feed forward artificial neural network.

At least one variable quantity of at least one of the plurality of machine learning input parameters may have been imputed during generating the machine learning model.

The machine learning model may be updated using the glucose measurement values of the user and the predicted risk and/or first risk and/or second risk. Alternatively, the machine learning model may be static / fixed with respect to predicted risks for the user. In other words, the machine learning model may be generated using historical glucose monitoring data of the user population only.

The method may comprise, before providing the user values, verifying whether at least one of a plurality of triggering conditions are met, the plurality of triggering conditions comprising at least one of:

- the cut-off time being within an allowable cut-off time range,
- a latest carbohydrate intake being before a first waiting period before the cut-off time,
- a latest insulin intake being before a second waiting period before the cut-off time,
- a previous risk prediction being not later than a third waiting period before the cut-off time, and
- no hypoglycemic event detected within a fourth waiting period before the cut-off time.

The allowable cut-off time range may for example be from 8 pm to 4 am, preferably from 9 pm to 2 am. The first waiting period may have a length between 10 minutes and 30 minutes, preferably 20 minutes. The second waiting period may have a length between 10 minutes and 30 minutes, preferably 20 minutes. The third waiting period may have a length between 10 minutes and 30 minutes, preferably 20 minutes. The fourth waiting period may have a length between 10 minutes and 30 minutes, preferably 20 minutes.

The method may be aborted and/or an error notification be generated and/or displayed based on at least one of a plurality of triggering conditions not being met.

The method may comprise, in particular before predicting the risk, verifying whether at least one of, preferably each of, the user values for the plurality of machine learning input parameters is within a (predefined) allowable range. The method may be aborted and/or an error notification be generated and/or displayed based on at least one of the user values not being within the predefined allowable range.

For example, the allowable range for the at least one first glucose measurement value may be from 70 mg/dL to 400 mg/dL.

The method may comprise (re-)calibrating, in particular before generating the output and/or after predicting the risk, the predicted risk to conform to an expected distribution of probabilities. Calibrating the predicted risk may in particular ensure that the predicted risk is between 0 and 1. Calibrating the predicted risk may comprise transforming the risk (and/or a corresponding probability) using a linear function. For example, the predicted risk may be calibrated using $f(p) = ap + b$, with predicted risk $p$, slope $a$, offset $b$, and calibrated risk $f(p)$. The linear function may be determined from a plurality of previously predicted risks using the machine learning model.

The method may comprise verifying whether at least one of the predicted risk, the first risk, and the second risk and/or the respective corresponding probability is within a (predefined) allowable probability range. The allowable probability range may be from 0 to 1.

Within the context of the present disclosure, the indication of an interval or a parameter range using the terms "between ... and" includes the limit points of the interval or parameter range.

The embodiments described above in connection with the computer-implemented method for predicting a risk whether a hypoglycemic event will occur during a prediction time interval may be provided correspondingly for the data processing system, the (CGM) system, and the remote control. In particular, any of the preceding method steps may be carried out in the data processing system and/or the CGM system, in particular in at least one of the user device, the glucose monitoring device, the remote control, and the medical server.

Description of further embodiments

In the following, embodiments, by way of example, are described with reference to figures.

Fig. 1    shows a graphical representation of an arrangement including a CGM system.
Fig. 2    shows a graphical representation of a method for predicting glucose values.
Fig. 3    shows a diagram including SHAP values for machine learning input parameters.
Fig. 4    shows calibration curves for a hypoglycemic event and for no hypoglycemic event.

Setup

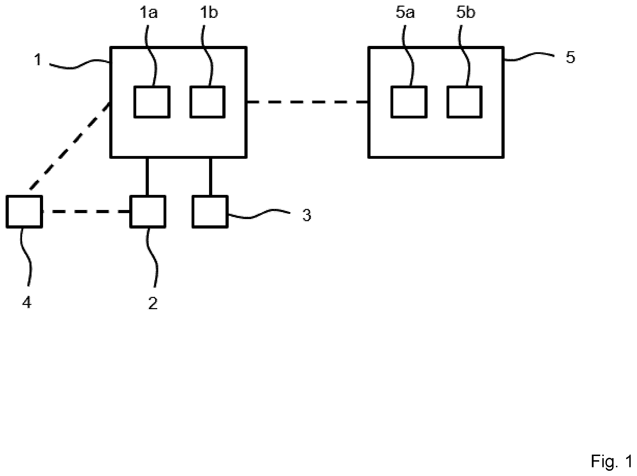

In Fig. 1, a graphical representation of an arrangement including a (CGM / continuous glucose monitoring) system is shown. The system comprises a user device 1, an input device 2, and an output device 3. Additionally, or alternatively, the system may comprise at least one of a data processing system, a glucose monitoring device / (continuous) glucose measurement device 4, a medical server 5, a remote control, and a processing unit. The data processing system may be one of the user device 1, the glucose monitoring device 4, and the medical server 5. The user device 1 may be the remote control.

The user device 1 comprises one or more processors 1a and a memory 1b for storing (machine readable) instructions. The user device 1 is connected to an input device 2 configured to receive (user) input data and an output device 3 configured for outputting data. The input device 2 and/or the output device 3 may or may not be implemented integrally with the user device 1. In particular, the user device 1 may or may not comprise the input device 2 and/or the output device 3. The user device 1 may be a mobile phone, a portable digital assistant (PDA), a mobile computing device such as a laptop, a tablet, or a smart phone. The one or more processors 1a may be a controller, an integrated circuit, a microchip, a computer, or any other computing device capable of executing machine readable instructions. The memory 1b may be RAM, ROM, a flash memory, a hard drive, or any device capable of storing machine readable instructions.

The output device 3 may be configured to provide graphical, textual and/or auditory information. The output device 3 may include an electronic display such as, for example, a liquid crystal display, a thin film transistor display, a light emitting diode display, a touch screen, or any other device capable of transforming signals from a processor into an optical output, or a mechanical output, such as, for example, a speaker or a printer for displaying information.

The user device 1 and/or the input device 2 may comprise or be coupled to a (continuous) glucose monitoring device 4 and/or a (continuous) glucose monitoring system 4 coupled to a person having diabetes. The input device 2 may be configured to receive glucose measurement values of the user. The input device 2 may be further configured to transmit the glucose measurement values to the user device 1 a medical server 5.

The glucose monitoring device/system 4 may be configured to detect and/or measure glucose levels (e.g., glucose concentrations) and/or glucose measurement values, in particular when coupled to (e.g., worn by) the user. For example, the continuous glucose monitoring device 4 can be a disposable glucose sensor that is, e.g., worn under the skin.

Further, a medical server 5 communicatively coupled with and/or connected to the user device 1 and/or the input device 2 and/or the glucose monitoring device 4 is provided. The medical server 5 may comprise one or more processors 5a and a memory 5b.

Method steps

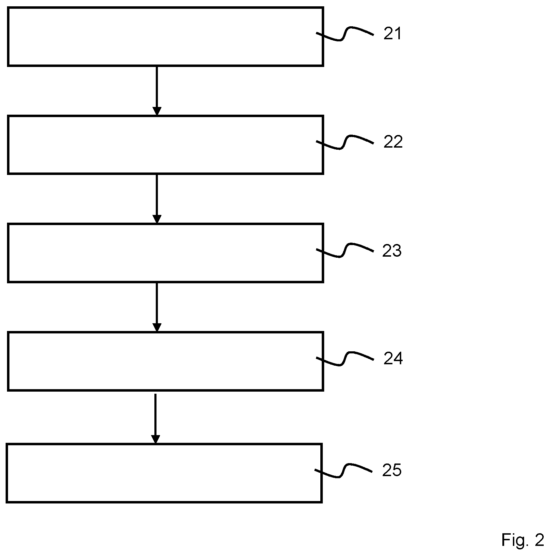

Fig. 2 shows a graphical representation of a computer-implemented method for predicting a risk for a hypoglycemic event. The method (in particular, its first step 21) may be triggered automatically at a predefined time of day or manually by the user, e.g., via the user device 1. The method may proceed if all triggering conditions are met, in particular, the cut-off time being within an allowable cut-off time range, a latest carbohydrate intake being before a first waiting period before the cut-off time, a latest insulin intake being before a second waiting period before the cut-off time, a previous risk prediction being not later than a third waiting period before the cut-off time, and no hypoglycemic event detected within a fourth waiting period before the cut-off time

In the first step 21, glucose monitoring data including glucose measurement values of a user are received in the user device 1 (from the glucose monitoring device 4). The glucose measurement values have been measured (by the glucose monitoring device 4 within an acquisition time interval) before a cut-off time. Preferably after each measurement, the corresponding measured glucose measurement value is transmitted from the glucose monitoring device 4 to the user device such as a smartphone. The glucose monitoring data may also be relayed from the glucose monitoring device 4 via the user device 1 to the medical server 5. Alternatively, the glucose monitoring data may be transmitted directly from the glucose monitoring device 4 to the medical server 5. Preferably the transmission between the devices and between the devices and the server is a wireless transmission, although a wired transmission is also feasible.

The glucose monitoring data, e.g., including glucose measurement values, insulin intake values, and carbohydrate intake values, are aggregated within a time grid of 5-minute time bins (buckets). Glucose measurement values assigned to the same time bin are averaged, yielding one glucose measurement value per time bin. Insulin intake values and carbohydrate intake values in the same time bin are summed up, yielding one insulin intake value / carbohydrate intake value per time bin.

In a second step 22, user values (specific value assignments) for a plurality of machine learning input parameters are determined from the glucose monitoring data, in particular, the aggregated values.

In an embodiment, the plurality of machine learning input parameters comprise:

- at least one first glucose measurement value determined from glucose measurements values measured within at most 10 minutes before the cut-off time ("CGM"),
- a hypoglycemic event share value indicative of a share of previous prediction time intervals with detected hypoglycemic events ("SLIDING_EVENT_HYPO_L1_BASELINE_ 28D_WP"),
- a first hypoglycemic event number value being a number of hypoglycemic events detected within previous prediction time intervals within a number of days before the cut-off time ranging from 7 days to 21 days ("HYPO_L1_START_CHEATING_PROFILE_SUM_ 14D_SUM_7H_SHIFT_POS_204"),
- a second hypoglycemic event number value being a number of hypoglycemic events detected within a detection time interval before the cut-off time having a length of at least 12 hours and at most 48 hours ("HYPO_L1_START_SUM_1D"),
- an insulin on board value predicted for an insulin on board target time after the cut-off time ("BOLUS_INSULIN_ON_BOARD"),
- a first slope value indicative of a change in time of a first set of glucose measurement values which have been measured within a first slope time interval before the cut-off time ("CGM_SLOPE_10M"),
- a second slope value indicative of a change in time of a second set of glucose measurement values which have been measured within a second slope time interval before the cut-off time ("CGM_SLOPE_30M"),
- an unexpected glucose drop value being a linear combination of a change in time of glucose measurement values, which have been measured within a glucose drop time interval before the cut-off time, and an insulin on board value at the cut-off time ("UNEXPECTED_CGM_FALL"), and
- a shifted time value determined by shifting the cut-off time into the future by at least 2 hours and at most 5 hours and rescaling the resulting value to a float interval [0, 24], wherein 0 corresponds to midnight and 24 to midnight next day ("TIME_OF_DAY_SHIFT_NEG_36").

In a particular example, at least one of the following machine learning input parameters (features) may be used:

| Feature name | Feature definition |
|---|---|
| CGM | Average CGM value in ]cut-off - 5 min, cut-off] |
| SLIDING_EVENT_HYPO_L1_BASELINE_ 28D_WP | Share of nighttime ranges with at least one level 1 hypoglycemic event out of all nighttime ranges within the last 28 nighttime ranges (excluding the current one) before the cut-off time. |
| HYPO_L1_START_CHEATING_PROFILE _SUM_14D_SUM_7H_SHIFT_POS_204 | Number of level 1 hypoglycemic events in the previous prediction time intervals of the last 14 days before the cut-off time. |
| HYPO_L1_START_SUM_1D | Number of level 1 hypoglycemic events in ]cut-off - 24 h, cut-off]. |

(continued)

| Feature name | Feature definition |
|---|---|
| BOLUS_INSULIN_ON_BOARD | Bolus insulin on board amount including all past insulin injections with an acting time of 240 minutes and a peak activity time of 60 minutes. |
| CGM_SLOPE_10M | Slope of CGM value in ]cut-off - 10 min, cut-off]. |
| CGM_SLOPE_30M | Slope of CGM value in ]cut-off - 30 min, cut-off]. |
| UNEXPECTED_CGM_FALL | CGM change in 1 hour + 50 * BOLUS_INSULIN_ON_BOARD at cut-off time. |
| TIME_OF_DAY_SHIFT_NEG_36 | Local time (time of the day) shifted in the future by 3 hours and rescaled into a float interval [0,24[. |

In a third step 23, each of the user values is checked as to being a float or an integer and each of the user values being within a predefined allowable range. Otherwise, the procedure is aborted.

In a fourth step 24, a risk whether a hypoglycemic event will occur during a prediction time interval (typically having a length of 7 hours) after the cut-off time (typically the time when the user requests a risk prediction) and associated with assumed sleep of the user is predicted based on the user values and using a machine learning model. To this end, an input vector comprising the user values for the machine learning input parameters is provided as input to the machine learning model. The machine learning model has been generated based on (historical user population values of) the machine learning input parameters.

In a particular embodiment, the machine learning model may provide a first probability value that an early hypoglycemic event will occur during a first sub interval of the prediction time interval, a second probability value that a late hypoglycemic event will occur during a second sub interval of the prediction time interval after the first sub interval, and a third probability value that no hypoglycemic event will occur during the prediction time interval.

Optionally, the probability values may be calibrated to conform to an expected distribution of probabilities. For example, the probability values may be calibrated using $f(p) = ap + b$, with predicted risk / probability value $p$, slope $a$, offset $b$, and calibrated risk / probability value $f(p)$. The linear function $f$ may be determined from a plurality of previously predicted risks using the machine learning model.

In a fifth step 25, an output is generated based on the predicted risk. The output may, e.g., comprise the predicted risk, a risk level determined from the predicted risk, a time information indicating a time range within which the hypoglycemic event may occur, a recommendation to the user indicating at least one user action to reduce the risk for a hypoglycemic event occurring during the prediction time interval, and a notification that no further user action is required at the moment. The output may be provided at the output device 3.

The output may be checked whether it conforms with predefined output requirements. In particular, each provided probability must be a value between 0 and 1. Further, the probabilities must add up to 1.

Evaluation

The performance of the machine learning model, in particular with respect to the machine learning input parameters / features, has been evaluated using SHAP (SHapley Additive exPlanations, cf. S. Lundberg et al., Nature machine intelligence, 2(1):56-67, 2020) values and synthetic data.

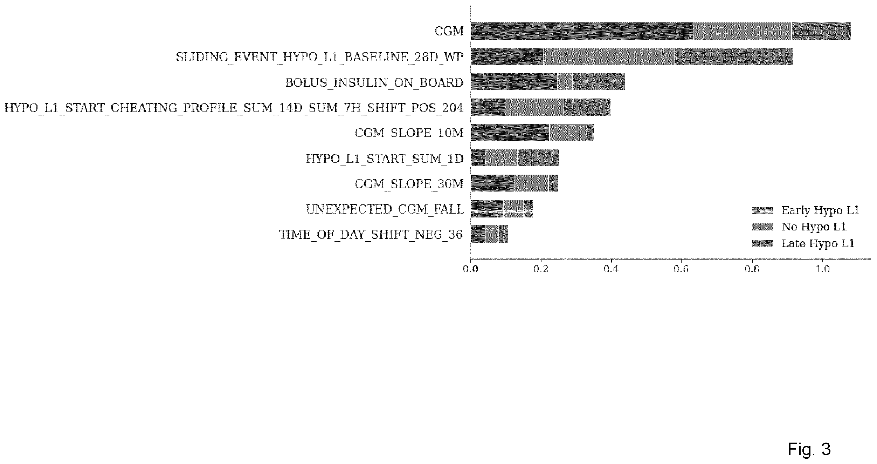

In Fig. 3, the respective SHAP values for the machine learning input parameters / features are shown. The length of each bar corresponds to the mean absolute SHAP value of the respective feature and illustrates the average impact on the model output. The importance for each of the classes "early hypoglycemic event", "no hypoglycemic event", and "late hypoglycemic event" is represented by using different shades of gray. In the present case, the SHAP values have been computed based on a testing partition of historical glucose monitoring data of a user population. SHAP values computed based on testing partitions yielded similar results. As shown in Fig. 3, the features "CGM" and "SLIDING_EVENT_HYPO_L1_BASELINE_ 28D_WP" have the greatest impact on risk prediction.

The effects of individual omitted features in prediction performance in terms of area under a receiver operating characteristics (ROC) curve (area under curve, AUC) are listed in the following table.

| Omitted feature | ROC AUC score | Relative change in ROC AUC score |
|---|---|---|
| No omitted feature | 0.8502 | 0.0 |
| BOLUS_INSULIN_ON_BOARD | 0.8398 | -0.0122 |
| CGM | 0.776 | -0.0873 |
| CGM_SLOPE_10M | 0.8434 | -0.008 |
| CGM_SLOPE_30M | 0.8435 | -0.0079 |
| HYPO_L1_START_CHEATING_PROFILE_ SUM_14D_SUM_7H_SHIFT_POS_204 | 0.8284 | -0.0256 |
| HYPO_L1_START_SUM_1D | 0.8388 | -0.0134 |
| SLIDING_EVENT_HYPO_L1_BASELINE_28D_WP | 0.8119 | -0.045 |
| TIME_OF_DAY_SHIFT_NEG_36 | 0.8462 | -0.0048 |
| UNEXPECTED_CGM_FALL | 0.8457 | -0.0053 |

As shown in the above table, the features "CGM" and "HYPO_L1_START_CHEATING_ PROFILE_SUM_14D_SUM_7H_SHIFT_POS_204" result in the largest performance drop.

The effect of cumulatively omitting features in the order of their impact on prediction performance in terms of ROC AUC (starting with the features with the smallest impact) is presented in the following table.

| Omitted features | ROC AUC score | Rel. change in ROC AUC score |
|---|---|---|
| TIME_OF_DAY_SHIFT_NEG_36, UNEXPECTED_CGM_FALL | 0.8431 | -0.0083 |
| TIME_OF_DAY_SHIFT_NEG_36, UNEXPECTED_CGM_FALL, CGM_SLOPE_30M | 0.8331 | -0.0202 |
| TIME_OF_DAY_SHIFT_NEG_36, UNEXPECTED_CGM_FALL, CGM_SLOPE_30M, CGM_SLOPE_10M | 0.8126 | -0.0443 |
| TIME_OF_DAY_SHIFT_NEG_36, UNEXPECTED_CGM_FALL, CGM_SLOPE_30M, CGM_SLOPE_10M, BOLUS_INSULIN_ON_BOARD | 0.8006 | -0.0584 |
| TIME_OF_DAY_SHIFT_NEG_36, UNEXPECTED_CGM_FALL, CGM_SLOPE_30M, CGM_SLOPE_10M, BOLUS_INSULIN_ON_BOARD, HYPO_L1_START_SUM_1D | 0.7879 | -0.0733 |
| TIME_OF_DAY_SHIFT_NEG_36, UNEXPECTED_CGM_FALL, CGM_SLOPE_30M, CGM_SLOPE_10M, BOLUS_INSULIN_ ON_BOARD, HYPO_L1_START_SUM_1D, HYPO_L1_START_ CHEATING_PROFILE_SUM_14D_SUM_7H_SHIFT_POS_204 | 0.7721 | -0.0918 |
| TIME_OF_DAY_SHIFT_NEG_36, UNEXPECTED_CGM_FALL, CGM_SLOPE_30M, CGM_SLOPE_10M, BOLUS_INSULIN_ON_BOARD, HYPO_L1_START_SUM_1D, HYPO_L1_START_CHEATING_PROFILE_SUM_14D_SUM_7H _SHIFT_POS_204, SLIDING_EVENT_HYPO_L1_BASELINE_28D_WP | 0.6955 | -0.182 |
| TIME_OF_DAY_SHIFT_NEG_36, UNEXPECTED_CGM_FALL, CGM_SLOPE_30M, CGM_SLOPE_10M, BOLUS_INSULIN_ON_BOARD, HYPO_L1_START_SUM_1D, HYPO_L1_START_ CHEATING_PROFILE_SUM_14D_SUM_7H_SHIFT_POS_204, SLIDING_EVENT_HYPO_L1_BASELINE_28D_WP, CGM | 0.5 | -0.4119 |

Hence, when omitting all features *except for* "CGM" and "SLIDING_EVENT_HYPO_L1_ BASELINE_28D_WP" (third to last line of the table), or in other words, using only the features "CGM" and "SLIDING_EVENT_HYPO_L1_BASELINE_28D_WP" in the machine learning model, the obtained ROC AUC score is 0.7721, which is substantially above a common AUC acceptability threshold of 0.7. Additionally including the features HYPO_L1_START_SUM_1D and HYPO_L1_START_CHEATING_PROFILE_SUM_14D_SUM_7H_SHIFT_POS_204 (and omitting the remaining listed features, line 4 of the table) yields an ROC AUC score of 0.8006, which is above 0.8.

In order to check whether the probabilities predicted by the machine learning model match the expected distribution of probabilities (the predicted probabilities being calibrated), the mean probability as a function of the fraction of positives has been determined.

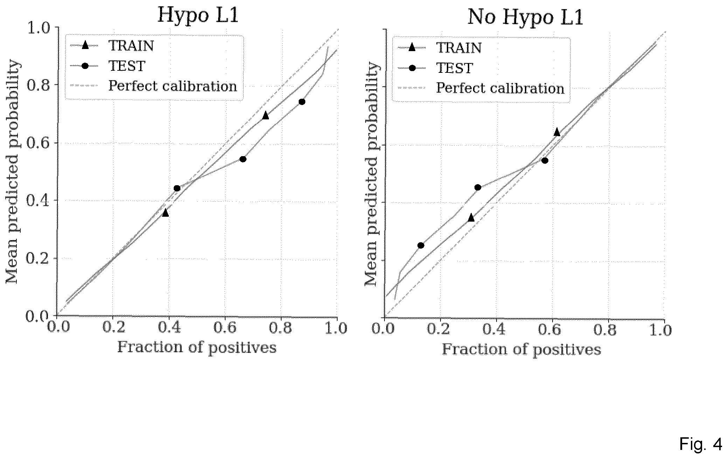

In Fig. 4, calibration curves for a hypoglycemic event and for no hypoglycemic event using both training data and testing data in comparison with an ideal ("perfect") calibration curve are shown. As illustrated by the figure, the model can be considered as already well-calibrated. (Re-)calibration for the overall model is thus optional.

The features disclosed in this description, the drawings and/or the claims may be material for the realization of various embodiments, taken in isolation or in various combinations thereof.

**Claims**

1. A computer-implemented method for predicting a risk whether a hypoglycemic event will occur during a prediction time interval, comprising:

    - providing user values for a plurality of machine learning input parameters, the user values determined from glucose monitoring data with glucose measurement values detected for a user before a cut-off time, the plurality of machine learning input parameters comprising:

        - at least one first glucose measurement value determined from glucose measurements values measured within at most 10 minutes before the cut-off time,
        - a hypoglycemic event share value indicative of a fraction of previous prediction time intervals with detected hypoglycemic events and a total number of previous prediction time intervals before the cut-off time, and
        - a first hypoglycemic event number value being a first number of hypoglycemic events detected within the previous prediction time intervals within a number of days before the cut-off time ranging from 7 days to 21 days;

    - predicting, based on the user values and using a machine learning model, a risk whether a hypoglycemic event will occur during a prediction time interval associated with assumed sleep of the user, the machine learning model generated based on the machine learning input parameters and using historical glucose monitoring data of a user population; and
    - generating an output based on the predicted risk.

2. The method of claim 1, wherein the plurality of machine learning input parameters further comprises:

    - a second hypoglycemic event number value being a second number of hypoglycemic events detected within a detection time interval before the cut-off time having a length of at least 12 hours and at most 48 hours.

3. The method of claim 1 or 2, wherein the plurality of machine learning input parameters further comprises an insulin on board value predicted for an insulin on board target time after the cut-off time.

4. The method of any of the preceding claims, wherein the plurality of machine learning input parameters further comprises at least one of:

    - a first slope value indicative of a change in time of a first set of glucose measurement values which have been measured within a first slope time interval before the cut-off time and
    - a second slope value indicative of a change in time of a second set of glucose measurement values which have been measured within a second slope time interval before the cut-off time.

5. The method of any of the preceding claims, wherein the plurality of machine learning input parameters further comprises at least one of:

    - an unexpected glucose drop value being a linear combination of a change in time of glucose measurement values, which have been measured within a glucose drop time interval before the cut-off time, and an insulin on board value at the cut-off time,

- a shifted time value determined by shifting the cut-off time into the future by at least 2 hours and at most 5 hours and rescaling the resulting value to a float interval, and
- a carbohydrate consumption by the user within a carbohydrate consumption interval before the cut-off time.

6. The method of any of the preceding claims, wherein the prediction time interval is a nighttime interval.

7. The method of any of the preceding claims, wherein the prediction time has a length between 5 hours and 10 hours.

8. The method of any of the preceding claims, wherein predicting the risk comprises predicting a first risk whether an early hypoglycemic event will occur during a first sub interval of the prediction time interval and predicting a second risk whether a late hypoglycemic event will occur during a second sub interval of the prediction time interval after the first sub interval.

9. The method of any of the preceding claims, wherein the output comprises at least one of:

   - the predicted risk,
   - a risk level determined from the predicted risk,
   - a time information indicating a time range within which the hypoglycemic event may occur,
   - a recommendation indicating at least one user action to reduce the risk for a hypoglycemic event to occur during the prediction time interval, and
   - a notification indicating that no further user action is required at the moment.

10. The method of any of the preceding claims, wherein the machine learning model has been generated using at least one of gradient boosting, a decision tree, a random forest, a logistic regression, and an artificial neural network.

11. The method of any of the preceding claims, wherein the machine learning model is updated using the glucose measurement values of the user and the predicted risk.

12. The method of any of the preceding claims, further comprising verifying, before providing the user values, whether at least one of a plurality of triggering conditions are met, the plurality of triggering conditions comprising at least one of:

   - the cut-off time being within an allowable cut-off time range,
   - a latest carbohydrate intake being before a first waiting period before the cut-off time,
   - a latest insulin intake being before a second waiting period before the cut-off time,
   - a previous risk prediction being not later than a third waiting period before the cut-off time, and
   - no hypoglycemic event detected within a fourth waiting period before the cut-off time.

13. The method of any of the preceding claims, further comprising calibrating the predicted risk to conform to an expected distribution of probabilities.

14. A data processing system comprising means for carrying out the method of any of the preceding claims.

15. A computer program comprising instructions which, when the computer program is executed by a data processing system, cause the data processing system to carry out the method of any of claims 1 to 13.

16. A system comprising a continuous glucose measurement sensor device (4), a remote control configured to receive glucose data from the continuous glucose measurement sensor device (4), and a processing unit configured to carry out the method according to any of claims 1 to 13.

17. A remote control configured to generate an output and comprising a processor configured to carry out the method according to any of claims 1 to 13.


**Patentansprüche**

1. Computerimplementiertes Verfahren zur Vorhersage eines Risikos, ob ein hypoglykämisches Ereignis während eines Vorhersagezeitintervalls auftritt, umfassend:

- Bereitstellen von Benutzerwerten für eine Mehrzahl von Eingabeparametern für maschinelles Lernen, wobei die Benutzerwerte aus Glukose-Überwachungsdaten mit Glukosemesswerten bestimmt werden, die für einen Benutzer vor einer Cut-off-Zeit erfasst wurden, wobei die Mehrzahl von Eingabeparametern für maschinelles Lernen Folgendes umfasst:

    - mindestens einen ersten Glukosemesswert, der aus Glukosemesswerten bestimmt wird, die innerhalb von höchstens 10 Minuten vor der Cut-off-Zeit gemessen wurden,
    - einen hypoglykämischen Ereignisanteilswert, der einen Bruchteil von vorherigen Vorhersagezeitintervallen mit erfassten hypoglykämischen Ereignissen und einer Gesamtzahl von vorherigen Vorhersagezeitintervallen vor der Cut-off-Zeit angibt, und
    - einen ersten Zahlenwert für ein hypoglykämisches Ereignis, der eine erste Anzahl von hypoglykämischen Ereignissen ist, die innerhalb der vorherigen Vorhersagezeitintervalle innerhalb einer Anzahl von Tagen vor der Cut-off-Zeit im Bereich von 7 Tagen bis 21 Tagen erfasst wurden;

- Vorhersagen eines Risikos, basierend auf den Benutzerwerten und unter Verwendung eines Maschinelles-Lernen-Modells, ob ein hypoglykämisches Ereignis während eines Vorhersagezeitintervalls auftritt, das mit vermutetem Schlaf des Benutzers assoziiert ist, wobei das Maschinelles-Lernen-Modell basierend auf den Eingabeparametern für maschinelles Lernen und unter Verwendung von historischen Glukose-Überwachungsdaten einer Benutzerpopulation erzeugt wurde; und
- Erzeugen einer Ausgabe basierend auf dem vorhergesagten Risiko.

2. Verfahren nach Anspruch 1, wobei die Mehrzahl von Eingabeparametern für maschinelles Lernen ferner Folgendes umfasst:

    - einen zweiten Zahlenwert für ein hypoglykämisches Ereignis, der eine zweite Anzahl von hypoglykämischen Ereignissen ist, die innerhalb eines Erfassungszeitintervalls vor der Cut-off-Zeit mit einer Länge von mindestens 12 Stunden und höchstens 48 Stunden erfasst wurden.

3. Verfahren nach Anspruch 1 oder 2, wobei die Mehrzahl von Eingabeparametern für maschinelles Lernen ferner einen Insulin-on-Board-Wert umfasst, der für eine Insulin-on-Board-Zielzeit nach der Cut-off-Zeit vorhergesagt wird.

4. Verfahren nach einem der vorhergehenden Ansprüche, wobei die Mehrzahl von Eingabeparametern für maschinelles Lernen ferner mindestens eines von Folgendem umfasst:

    - einen ersten Steigungswert, der eine Änderung eines ersten Satzes von Glukosemesswerten, die innerhalb eines ersten Steigungszeitintervalls vor der Cut-off-Zeit gemessen wurden, im Laufe der Zeit angibt, und
    - einen zweiten Steigungswert, der eine Änderung eines zweiten Satzes von Glukosemesswerten, die innerhalb eines zweiten Steigungszeitintervalls vor der Cut-off-Zeit gemessen wurden, im Laufe der Zeit angibt.

5. Verfahren nach einem der vorhergehenden Ansprüche, wobei die Mehrzahl von Eingabeparametern für maschinelles Lernen ferner mindestens eines von Folgendem umfasst:

    - einen Wert für unerwarteten Glukoseabfall, der eine lineare Kombination aus einer Änderung von Glukosemesswerten im Laufe der Zeit, die innerhalb eines Glukoseabfall-Zeitintervalls vor der Cut-off-Zeit gemessen wurden, und einem Insulin-on-Board-Wert zur Cut-off-Zeit ist,
    - einen verschobenen Zeitwert, der durch Verschieben der Cut-off-Zeit um mindestens 2 Stunden und höchstens 5 Stunden in die Zukunft und Neuskalieren des resultierenden Werts auf ein Float-Intervall bestimmt wird, und
    - einen Kohlenhydratverbrauch durch den Benutzer innerhalb eines Kohlenhydratverbrauchsintervalls vor der Cut-off-Zeit.

6. Verfahren nach einem der vorhergehenden Ansprüche, wobei das Vorhersagezeitintervall ein Nachtzeitintervall ist.

7. Verfahren nach einem der vorhergehenden Ansprüche, wobei die Vorhersagezeit eine Länge zwischen 5 Stunden und 10 Stunden aufweist.

8. Verfahren nach einem der vorhergehenden Ansprüche, wobei das Vorhersagen des Risikos das Vorhersagen eines ersten Risikos, ob ein frühes hypoglykämisches Ereignis während eines ersten Unterintervalls des Vorhersagezeitintervalls auftritt, und das Vorhersagen eines zweiten Risikos, ob ein spätes hypoglykämisches Ereignis während eines zweiten Unterintervalls des Vorhersagezeitintervalls nach dem ersten Unterintervall auftritt, umfasst.

9. Verfahren nach einem der vorhergehenden Ansprüche, wobei die Ausgabe mindestens eines von Folgendem umfasst:

   - das vorhergesagte Risiko,
   - ein Risikoniveau, das aus dem vorhergesagten Risiko bestimmt wird,
   - eine Zeitinformation, die einen Zeitbereich angibt, innerhalb dessen das hypoglykämische Ereignis auftreten kann,
   - eine Empfehlung, die mindestens eine Benutzermaßnahme angibt, um das Risiko zu verringern, dass ein hypoglykämisches Ereignis während des Vorhersagezeitintervalls auftritt, und
   - eine Benachrichtigung, die angibt, dass momentan keine weitere Benutzermaßnahme erforderlich ist.

10. Verfahren nach einem der vorhergehenden Ansprüche, wobei das Modell für maschinelles Lernen unter Verwendung von mindestens einem aus Gradientenverstärkung, einem Entscheidungsbaum, einem Zufallswald, einer logistischen Regression und einem künstlichen neuronalen Netzwerk erzeugt wurde.

11. Verfahren nach einem der vorhergehenden Ansprüche, wobei das Maschinelles-Lernen-Modell unter Verwendung der Glukosemesswerte des Benutzers und des vorhergesagten Risikos aktualisiert wird.

12. Verfahren nach einem der vorhergehenden Ansprüche, ferner umfassend ein Verifizieren, ob mindestens eine aus einer Mehrzahl von Auslösebedingungen erfüllt ist, vor dem Bereitstellen der Benutzerwerte, wobei die Mehrzahl von Auslösebedingungen mindestens eines von Folgendem umfasst:

   - die Cut-off-Zeit liegt innerhalb eines zulässigen Cut-off-Zeitbereichs,
   - eine letzte Kohlenhydratzufuhr findet vor einer ersten Wartezeit vor der Cut-off-Zeit statt,
   - eine letzte Insulinzufuhr findet vor einer zweiten Wartezeit vor der Cut-off-Zeit statt,
   - eine vorherige Risikovorhersage findet nicht später als nach einer dritten Wartezeit vor der Cut-off-Zeit statt und
   - kein hypoglykämisches Ereignis, das innerhalb einer vierten Wartezeit vor der Cut-off-Zeit erfasst wurde.

13. Verfahren nach einem der vorhergehenden Ansprüche, ferner umfassend das Kalibrieren des vorhergesagten Risikos, um einer erwarteten Verteilung von Wahrscheinlichkeiten zu entsprechen.

14. Datenverarbeitungssystem, umfassend Mittel zum Ausführen des Verfahrens nach einem der vorhergehenden Ansprüche.

15. Computerprogramm, umfassend Anweisungen, die, wenn das Computerprogramm von einem Datenverarbeitungssystem ausgeführt wird, das Datenverarbeitungssystem veranlassen, das Verfahren nach einem der Ansprüche 1 bis 13 auszuführen.

16. System, umfassend eine Sensorvorrichtung zur kontinuierlichen Glukosemessung (4), eine Fernsteuerung, die eingerichtet ist, Glukosedaten von der Sensorvorrichtung zur kontinuierlichen Glukosemessung (4) zu empfangen, und eine Verarbeitungseinheit, die eingerichtet ist, das Verfahren nach einem der Ansprüche 1 bis 13 auszuführen.

17. Fernsteuerung, die eingerichtet ist, eine Ausgabe zu erzeugen, und die einen Prozessor umfasst, der eingerichtet ist, das Verfahren nach einem der Ansprüche 1 bis 13 auszuführen.

**Revendications**

1. Procédé mis en œuvre par ordinateur pour prédire un risque d'apparition d'un événement hypoglycémique pendant un intervalle de temps de prédiction, comprenant :

   - la fourniture de valeurs d'utilisateur pour une pluralité de paramètres d'entrée d'apprentissage automatique, les valeurs d'utilisateur étant déterminées à partir de données de surveillance du glucose avec des valeurs de mesure de glucose détectées pour un utilisateur avant un temps seuil, la pluralité de paramètres d'entrée d'apprentissage automatique comprenant :

- au moins une première valeur de mesure de glucose déterminée à partir de valeurs de mesure de glucose mesurées pendant au plus 10 minutes avant le temps seuil,
- une valeur de partage d'événement hypoglycémique indiquant une fraction d'intervalles de temps de prédiction précédents avec des événements hypoglycémiques détectés et un nombre total d'intervalles de temps de prédiction précédents avant le temps seuil, et
- une première valeur de nombre d'événements hypoglycémiques étant un premier nombre d'événements hypoglycémiques détectés pendant les intervalles de temps de prédiction précédents pendant un certain nombre de jours avant le temps seuil compris dans la plage allant de 7 jours à 21 jours ;

- la prédiction, sur la base des valeurs d'utilisateur et à l'aide d'un modèle d'apprentissage automatique, d'un risque d'apparition d'un événement hypoglycémique pendant un intervalle de temps de prédiction associé à un sommeil présumé de l'utilisateur, le modèle d'apprentissage automatique étant généré sur la base des paramètres d'entrée d'apprentissage automatique et à l'aide des données historiques de surveillance de glucose d'une population d'utilisateurs ; et
- la génération d'une sortie sur la base du risque prédit.

2. Procédé selon la revendication 1, dans lequel la pluralité de paramètres d'entrée d'apprentissage automatique comprend en outre :

- une seconde valeur de nombre d'événements hypoglycémiques qui est un second nombre d'événements hypoglycémiques détectés pendant un intervalle de temps de détection avant le temps seuil ayant une longueur d'au moins 12 heures et d'au plus 48 heures.

3. Procédé selon la revendication 1 ou 2, dans lequel la pluralité de paramètres d'entrée d'apprentissage automatique comprend en outre une valeur d'insuline active prédite pour un temps cible d'insuline active après le temps seuil.

4. Procédé selon l'une quelconque des revendications précédentes, dans lequel la pluralité de paramètres d'entrée d'apprentissage automatique comprend en outre au moins l'une parmi :

- une première valeur de pente indiquant une variation dans le temps d'un premier ensemble de valeurs de mesure de glucose qui ont été mesurées pendant un premier intervalle de temps de pente avant le temps seuil et
- une seconde valeur de pente indiquant une variation dans le temps d'un second ensemble de valeurs de mesure de glucose qui ont été mesurées pendant un second intervalle de temps de pente avant le temps seuil.

5. Procédé selon l'une quelconque des revendications précédentes, dans lequel la pluralité de paramètres d'entrée d'apprentissage automatique comprend en outre au moins l'une parmi :

- une valeur de chute de glucose inattendue qui est une combinaison linéaire d'une variation dans le temps de valeurs de mesure de glucose, qui ont été mesurées pendant un intervalle de temps de chute de glucose avant le temps seuil, et une valeur d'insuline active au temps seuil,
- une valeur de temps décalé déterminée par décalage du temps seuil dans le futur d'au moins 2 heures et d'au plus 5 heures et mise à l'échelle de la valeur résultante par rapport à un intervalle flottant, et
- une consommation de glucides par l'utilisateur au sein d'un intervalle de consommation de glucides avant le temps seuil.

6. Procédé selon l'une quelconque des revendications précédentes, dans lequel l'intervalle de temps de prédiction est un intervalle nocturne.

7. Procédé selon l'une quelconque des revendications précédentes, dans lequel le temps de prédiction a une longueur entre 5 heures et 10 heures.

8. Procédé selon l'une quelconque des revendications précédentes, dans lequel la prédiction du risque comprend la prédiction d'un premier risque d'apparition d'un événement hypoglycémique précoce pendant un premier sous-intervalle de l'intervalle de temps de prédiction et la prédiction d'un second risque d'apparition d'un événement hypoglycémique tardif pendant un second sous-intervalle de l'intervalle de temps de prédiction après le premier sous-intervalle.

9. Procédé selon l'une quelconque des revendications précédentes, dans lequel la sortie comprend au moins l'un parmi :

- le risque prédit,
- un niveau de risque déterminé à partir du risque prédit,
- des informations temporelles indiquant une plage de temps au sein de laquelle peut apparaître l'événement hypoglycémique,
- une recommandation indiquant au moins une action de l'utilisateur pour réduire le risque d'apparition d'un événement hypoglycémique pendant l'intervalle de temps de prédiction, et
- une notification indiquant qu'aucune autre action de l'utilisateur n'est requise pour le moment.

10. Procédé selon l'une quelconque des revendications précédentes, dans lequel le modèle d'apprentissage automatique a été généré à l'aide d'au moins l'un parmi une amplification de gradient, un arbre de décision, une forêt aléatoire, une régression logistique et un réseau de neurones artificiels.

11. Procédé selon l'une quelconque des revendications précédentes, dans lequel le modèle d'apprentissage automatique est mis à jour à l'aide des valeurs de mesure de glucose de l'utilisateur et du risque prédit.

12. Procédé selon l'une quelconque des revendications précédentes, comprenant en outre la vérification, avant la fourniture des valeurs d'utilisateur, si au moins l'une d'une pluralité de conditions de déclenchement sont satisfaites, la pluralité de conditions de déclenchement comprenant au moins l'un parmi :

- le temps seuil qui est dans une plage de temps seuil autorisée,
- un dernier apport en glucides qui est avant une première période d'attente avant le temps seuil,
- un dernier apport en insuline qui est avant une deuxième période d'attente avant le temps seuil,
- une précédente prédiction de risque qui n'est pas postérieure à une troisième période d'attente avant le temps seuil, et
- aucun événement hypoglycémique détecté pendant une quatrième période d'attente avant le temps seuil.

13. Procédé selon l'une quelconque des revendications précédentes, comprenant en outre l'étalonnage du risque prédit pour se conformer à une répartition des probabilités attendue.

14. Système de traitement de données comprenant des moyens pour réaliser le procédé selon l'une quelconque des revendications précédentes.

15. Programme informatique comprenant des instructions qui, lorsque le programme informatique est exécuté par un système de traitement de données, amènent le système de traitement de données à réaliser le procédé selon l'une quelconque des revendications 1 à 13.

16. Système comprenant un dispositif capteur de mesure de glucose en continu (4), une télécommande configurée pour recevoir les données de glucose en provenance du dispositif capteur de mesure de glucose en continu (4) et une unité de traitement configurée pour réaliser le procédé selon l'une quelconque des revendications 1 à 13.

17. Commande à distance configurée pour générer une sortie et comprenant un processeur configuré pour réaliser le procédé selon l'une quelconque des revendications 1 à 13.

EP 4 614 504 B1

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11350876 B2
- US 20220061710 A1
- US 20220142521 A1
- US 20210338116 A1
- US 2020375549 A1

**Non-patent literature cited in the description**

- **S. LUNDBERG et al.** *Nature machine intelligence*, 2020, vol. 2 (1), 56-67